# EUROPEAN PATENT SPECIFICATION

(11) **EP 3 365 108 B1**
(45) Date of publication and mention of the grant of the patent: **12.06.2024**
(21) Application number: 16858226.0
(22) Date of filing: 20.10.2016
(51) Int. Cl.: B01L 3/00, C08L 83/04, C08L 83/10, C08G 77/04, C08G 77/44

(54) **FILLER FLUID FOR FLUIDIC DEVICES**
FÜLLFLUID FÜR FLUIDISCHE VORRICHTUNGEN
FLUIDE DE REMPLISSAGE POUR DISPOSITIFS FLUIDIQUES

(30) Priority: 22.10.2015 US 201562245147 P
(43) Date of publication of application: 29.08.2018
(73) Proprietor: Illumina, Inc., San Diego, CA 92122 (US); Illumina Cambridge Limited, Cambridge CB21 6DF (GB)
(72) Inventor: VON HATTEN, Xavier, San Diego, CA 92122 (US); PERBOST, Michel, San Diego, CA 92122 (US); HUANG, Heng, San Diego, CA 92122 (US); LEE, Nicole, San Diego, CA 92122 (US); LAM, Vicky, V., San Diego, CA 92122 (US); JUAN, Nilda, San Diego, CA 92122 (US); MERKEL, Timothy, San Diego, CA 92122 (US); BEIERLE, John, M., San Diego, CA 92122 (US)
(74) Representative: Murgitroyd & Company
(86) International application number: PCT/US2016/057941
(87) International publication number: WO 2017/070363

(56) References cited:
- EP-A1- 0 635 526
- WO-A1-2016/164592
- WO-A2-2009/135205
- US-A- 4 463 118
- US-A- 5 385 730
- US-A1- 2004 234 475
- US-A1- 2011 303 542
- US-A1- 2014 190 830
- US-A1- 2014 231 259
- US-A1- 2015 075 986
- US-B1- 6 174 968
- ERK, KENDRA A. ET AL.: 'Shear and dilational interfacial rheology of surfactant-stabilized droplets.' JOURNAL OF COLLOID AND INTERFACE SCIENCE vol. 377, 2012, pages 442 - 449, XP028485539
- MEHTA, SOMIL C. ET AL.: 'Mechanism of stabilization of silicone oil-water emulsions using hybrid siloxane polymers.' LANGMUIR vol. 24, 2008, pages 4558 - 4563, XP055378279

## Description

### BACKGROUND

Microfluidic devices are miniature fluidic devices dealing with small fluidic volumes, usually in the sub-milliliter range. Microfluidic devices may have micromechanical structures (microchannels, microtracks, micropaths, microvalves and others) and employ various fluid-moving mechanisms, such as mechanical parts (e.g., micropumps) hydropneumatic devices/methods and electrically-based effects (electrophoretic, dielectrophoretic, electro-osmotic, electrowetting, opto-electrowetting, and variations of these effects as well as other effects). WO2016/164592A1 discloses fluidic devices using siloxane block copolymer CMS-222.

### SUMMARY

The present invention is defined in the appended claims. Any disclosure lying outside the scope of the invention is only intended for illustrative, informative as well as comparative purposes.

### BRIEF DESCRIPTION OF THE DRAWINGS

**FIGS. 1A** and **1B** show experimental data on the interfacial tension (IFT) of filler fluids containing CMS-222 against Reagent A (BBS) and Reagent B (ESL) reagents and observable process capability improvement with a filler fluid that contained the CMS-222 siloxane block co-polymer.
**FIG. 2** shows experimental data demonstrating that the working range for Span^{®} 85 is 0.0015% - 0.004%, and the working range for CMS-222 is 0.02% - 0.1%.
**FIG. 3** shows experimental data of the IFT of CMS-222 against a variety of standard reagents.
**FIG. 4** shows, in one embodiment, experimental data of the IFT of different siloxane block co-polymers against Reagent A (BBS) and Reagent B (ESL) reagents.

### DETAILED DESCRIPTION

The present disclosure describes compositions and methods for improving droplet operations in microfluidic devices. For biomedical applications, some microfluidic devices are designed to conduct sample processing, including concentration, filtration, washing, dispensing, mixing, transport, sample splitting, sample lysing and other sample handling functions.

Microfluidic devices may include digital fluidic cartridges having a top plate, usually made of plastic, which is coated with a conductive coating layer, two hydrophobic layers with tracks or paths of electrode in between, a dielectric coating and a printed circuit board (PCB) bottom. The space between the two hydrophobic layers can be filled with a filler fluid which is immiscible or substantially immiscible with the sample fluid. In some embodiments the sample fluid uses electrowetting to move a sample through the filler fluid within the microfluidic device.

As used herein one fluid is immiscible in another fluid if they do not form a homogenous mixture when added together. Fluids that are immiscible will separate into different liquid layers. The term "substantially immiscible" as used herein refers to a fluid which, when mixed with a droplet phase, will almost completely separate into two discrete phases after equilibration, with only an insignificant portion of one fluid being mixed with the other fluid. For example, a substantially immiscible mixture may have a volume fraction of a first liquid that is less than about 0.5%, about 0.3%, about 0.1%, about 0.05% or about 0.01% miscible with a second fluid.

The present disclosure discusses fluidic devices such as digital microfluidic devices and methods of improving droplets operation, sample analysis, devices life-span and robustness in fluidic devices that use a filler fluid. Electrowetting devices may comprise a hydrophobic filler fluid within the device and a hydrophilic aqueous sample mixed within a predetermined buffer that forms a sample droplet in the filler fluid. For the droplet to move efficiently within the device, a droplet surface tension of about 6-12 dynes/cm is normally desired in some embodiments. This target surface tension may be achieved by adding a surfactant in the buffer. However, for some types of assays the surfactant may negatively affect the assay operation, for example by inhibiting particular chemical reactions. Thus, the present invention provides improved filler fluids that contain a siloxane-based block co-polymer hydrophobic surfactant. In some embodiments, the siloxane-based block co-polymer surfactant can adjust the surface tension of the sample droplet within the filler fluid to be within the target range of about 6 to about 12 dynes/cm.

In some embodiments, the filler fluid includes a low-viscosity oil such as a silicone oil. The low viscosity oil may have a viscosity of about 7 cSt or less, as one example. In embodiments that use a poly(dimethyl)siloxane (PDMS) based filler fluid, the polymer surfactant may be soluble in PDMS, but not in the aqueous buffer. One class of polymers, siloxane based block co-polymers, has been identified to be soluble in such filler fluids, and insoluble in aqueous buffers. Accordingly, one embodiment is a microfluidic device that uses a siloxane-based block co-polymer surfactant within a PDMS filler fluid. It should be appreciated that the filler fluids disclosed herein are compatible with biomedical fluidic applications.

In some embodiments, the siloxane based block co-polymer surfactant includes a siloxane backbone that is functionalized with a linear hydrophilic side chain, such as a hydrophilic head group.

### Definitions

Unless defined otherwise, all technical and scientific terms used herein have the same meaning as is commonly understood by one of ordinary skill in the art. The use of the term "including" as well as other forms, such as "include", "includes," and "included," is not limiting. The use of the term "having" as well as other forms, such as "have", "has," and "had," is not limiting. As used in this specification, whether in a transitional phrase or in the body of the claim, the terms "comprise(s)" and "comprising" are to be interpreted as having an open-ended meaning. That is, the above terms are to be interpreted synonymously with the phrases "having at least" or "including at least." For example, when used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

As used herein, the term "droplet actuator" means a device for manipulating droplets. For examples of droplet actuators, see Pamula et al., U.S. Patent No. 6,911,132, entitled "Apparatus for Manipulating Droplets by Electrowetting-Based Techniques," issued on June 28, 2005; Pamula et al., U.S. Patent Pub. No. 20060194331, entitled "Apparatuses and Methods for Manipulating Droplets on a Printed Circuit Board," published on August 31, 2006; Pollack et al., International Patent Pub. No. WO/2007/120241, entitled "Droplet-Based Biochemistry," published on October 25, 2007; Shenderov, U.S. Patent No. 6,773,566, entitled "Electrostatic Actuators for Microfluidics and Methods for Using Same," issued on August 10, 2004; Shenderov, U.S. Patent No. 6,565,727, entitled "Actuators for Microfluidics Without Moving Parts," issued on May 20, 2003; Kim et al., U.S. Patent Pub. No. 20030205632, entitled "Electrowetting-driven Micropumping," published on November 6, 2003; Kim et al., U.S. Patent Pub. No. 20060164490, entitled "Method and Apparatus for Promoting the Complete Transfer of Liquid Drops from a Nozzle," published on July 27, 2006; Kim et al., U.S. Patent Pub. No. 20070023292, entitled "Small Object Moving on Printed Circuit Board," published on February 1, 2007; Shah et al., U.S. Patent Pub. No. 20090283407, entitled "Method for Using Magnetic Particles in Droplet Microfluidics," published on November 19, 2009; Kim et al., U.S. Patent Pub. No. 20100096266, entitled "Method and Apparatus for Real-time Feedback Control of Electrical Manipulation of Droplets on Chip," published on April 22, 2010; Velev, U.S. Patent No. 7,547,380, entitled "Droplet Transportation Devices and Methods Having a Fluid Surface," issued on June 16, 2009; Sterling et al., U.S. Patent No. 7,163,612, entitled "Method, Apparatus and Article for Microfluidic Control via Electrowetting, for Chemical, Biochemical and Biological Assays and the Like," issued on January 16, 2007; Becker et al., U.S. Patent No. 7,641,779, entitled "Method and Apparatus for Programmable Fluidic Processing," issued on January 5, 2010; Becker et al., U.S. Patent No. 6,977,033, entitled "Method and Apparatus for Programmable Fluidic Processing," issued on December 20, 2005; Decre et al., U.S. Patent No. 7,328,979, entitled "System for Manipulation of a Body of Fluid," issued on February 12, 2008; Yamakawa et al., U.S. Patent Pub. No. 20060039823, entitled "Chemical Analysis Apparatus," published on February 23, 2006; Wu, U.S. Patent Pub. No. 20110048951, entitled "Digital Microfluidics Based Apparatus for Heat-exchanging Chemical Processes," published on March 3, 2011; Fouillet et al., U.S. Patent Pub. No. 20090192044, entitled "Electrode Addressing Method," published on July 30, 2009; Fouillet et al., U.S. Patent No. 7,052,244, entitled "Device for Displacement of Small Liquid Volumes Along a Micro-catenary Line by Electrostatic Forces," issued on May 30, 2006; Marchand et al., U.S. Patent Pub. No. 20080124252, entitled "Droplet Microreactor," published on May 29, 2008; Adachi et al., U.S. Patent Pub. No. 20090321262, entitled "Liquid Transfer Device," published on December 31, 2009; Roux et al., U.S. Patent Pub. No. 20050179746, entitled "Device for Controlling the Displacement of a Drop Between Two or Several Solid Substrates," published on August 18, 2005; and Dhindsa et al., "Virtual Electrowetting Channels: Electronic Liquid Transport with Continuous Channel Functionality," Lab Chip, 10:832-836 (2010).

Certain droplet actuators will include one or more substrates and electrodes associated with (e.g., layered on, attached to, and/or embedded in) the one or more substrates and arranged to conduct one or more droplet operations. In some cases two or more substrates are arranged with a droplet operations gap therebetween. For example, certain droplet actuators will include a base (or bottom) substrate, droplet operations electrodes associated with the substrate, one or more dielectric layers atop the substrate and/or electrodes, and optionally one or more hydrophobic layers atop the substrate, dielectric layers and/or the electrodes forming a droplet operations surface. A top substrate may also be provided, which is separated from the droplet operations surface by a gap, commonly referred to as a droplet operations gap. Various electrode arrangements on the top and/or bottom substrates are discussed in the above-referenced patents and applications and certain novel electrode arrangements are discussed in the description of the present disclosure. During droplet operations droplets may remain in continuous contact or frequent contact with a ground or reference electrode. A ground or reference electrode may be associated with the top substrate facing the gap or the bottom substrate facing the gap. Where electrodes are provided on both substrates, electrical contacts for coupling the electrodes to a droplet actuator instrument for controlling or monitoring the electrodes may be associated with one or both plates. In some cases, electrodes on one substrate are electrically coupled to the other substrate so that only one substrate is in contact with the droplet actuator. In one embodiment, a conductive material (e.g., an epoxy, such as MASTER BOND^{™} Polymer System EP79, available from Master Bond, Inc., Hackensack, NJ) provides the electrical connection between electrodes on one substrate and electrical paths on the other substrates, e.g., a ground electrode on a top substrate may be coupled to an electrical path on a bottom substrate by such a conductive material. Where multiple substrates are used, a spacer may be provided between the substrates to determine the height of the gap therebetween and define on-actuator dispensing reservoirs. The spacer height may, for example, be at least about 5 µm, about 100 µm, about 200 µm, about 250 µm, about 275 µm or more. Alternatively or additionally the spacer height may be at most about 600 µm, about 400 µm, about 350 µm, about 300 µm, or less. The spacer may, for example, be formed of a layer of projections form the top or bottom substrates, and/or a material inserted between the top and bottom substrates. One or more openings may be provided in the one or more substrates for forming a fluid path through which liquid may be delivered into the droplet operations gap. The one or more openings may in some cases be aligned for interaction with one or more electrodes, e.g., aligned such that liquid flowed through the opening will come into sufficient proximity with one or more droplet operations electrodes to permit a droplet operation to be effected by the droplet operations electrodes using the liquid. The base (or bottom) and top substrates may in some cases be formed as one integral component. One or more reference electrodes may be provided on the base (or bottom) and/or top substrates and/or in the gap. Examples of reference electrode arrangements are provided in the above referenced patents and patent applications.

For purposes of the present disclosure, the terms "layer" and "film" are used interchangeably to denote a structure or body that may be planar or substantially planar, and may be deposited on, formed on, coats, treats, or is otherwise disposed on another structure.

For purposes of the present disclosure, the term "communicate" (e.g., a first component "communicates with" or "is in communication with" a second component) is used herein to indicate a structural, functional, mechanical, electrical, optical, or fluidic relationship, or any combination thereof, between two or more components or elements. As such, the fact that one component is said to communicate with a second component is not intended to exclude the possibility that additional components may be present between, and/or operatively associated or engaged with, the first and second components.

For purposes of the present disclosure, it will be understood that when a given component such as a layer, region or substrate is referred to herein as being disposed or formed "on", "in", or "at" another component, that given component can be directly on the other component or, alternatively, intervening components (for example, one or more buffer layers, interlayers, electrodes or contacts) can also be present. It will be further understood that the terms "disposed on" and "formed on" are used interchangeably to describe how a given component is positioned or situated in relation to another component. Hence, the terms "disposed on" and "formed on" are not intended to introduce any limitations relating to particular methods of material transport, deposition, or fabrication.

For purposes of the present disclosure, it will be understood that when a liquid in any form (e.g., a droplet or a continuous body, whether moving or stationary) is described as being "on", "at", or "over" an electrode, array, matrix or surface, such liquid could be either in direct contact with the electrode, array, matrix or surface, or could be in contact with one or more layers or films that are interposed between the liquid and the electrode, array, matrix or surface.

As used herein, the term "reagent" describes any material useful for reacting with, diluting, solvating, suspending, emulsifying, encapsulating, interacting with, or adding to a sample material.

As used herein, the term "about", when modifying a numerical value, refers to variation in the numerical value that can occur. For example, variations can occur through liquid handling procedures used for making solutions; through inadvertent error in these procedures; through differences in the manufacture, source, or purity of the ingredients employed to make compositions or carry out methods. In one embodiment, the term "about" means within 1%, 5%, or up to 10% of the recited numerical value.

As used herein, "substantially" means to a great or significant extent. For example, one composition may be substantially the same as another composition when they are 95%, 96%, 97%, 98%, or 99% identical to one another.

### Filler Fluid

As used herein, the term "filler fluid" means a fluid that is associated with a fluidics device. The filler fluid may be used to fill the internal gaps of a fluidic, or microfluidic device, such as an electrowetting device. Within a fluidics device may be a droplet operations substrate that includes a droplet actuator for moving droplets within the device by electrical or electrowetting forces. The filler fluid may be substantially immiscible with a droplet phase of any aqueous sample placed within the fluidics device to render the droplet phase subject to electrode-mediated droplet operations. As one example, a droplet may be substantially immiscible with a filler fluid when less than about 0.1% of the volume fraction of the droplet liquid is miscible with the filler fluid liquid. Similarly, a silicone block co-polymer in a filler fluid may be substantially immiscible with a droplet liquid when less than about 0.1% of the volume fraction of the silicone block co-polymer in the filler fluid is miscible with the droplet liquid.

The droplet operations gap of a droplet actuator may be filled with a filler fluid. The filler fluid may, for example, be or include a low-viscosity oil, such as silicone oil or hexadecane filler fluid. The filler fluid may be or include a halogenated oil, such as a fluorinated or perfluorinated oil. The filler fluid may fill the entire gap of the droplet actuator or may coat one or more surfaces of the droplet actuator. Filler fluids may be conductive or non-conductive. Filler fluids may be selected to improve droplet operations and/or reduce loss of reagent or target substances from droplets, improve formation of microdroplets, reduce cross contamination between droplets, reduce contamination of droplet actuator surfaces, reduce degradation of droplet actuator materials, etc. For example, filler fluids may be selected for compatibility with droplet actuator materials. As an example, fluorinated filler fluids may be usefully employed with fluorinated surface coatings. Fluorinated filler fluids are useful to reduce loss of lipophilic compounds, such as umbelliferone substrates like 6-hexadecanoylamido-4-methylumbelliferone substrates (e.g., for use in Krabbe, Niemann-Pick, or other assays); other umbelliferone substrates are described in Winger et al., U.S. Patent Pub. No. 20110118132, entitled "Enzymatic Assays Using Umbelliferone Substrates with Cyclodextrins in Droplets of Oil," published on May 19, 2011. Examples of suitable fluorinated oils include those in the Galden line, such as Galden HT170 (bp = 170 °C, viscosity = 1.8 cSt, density = 1.77), Galden HT200 (bp = 200C, viscosity = 2.4 cSt, d = 1.79), Galden HT230 (bp = 230C, viscosity = 4.4 cSt, d = 1.82) (all from Solvay Solexis); those in the Novec line, such as Novec 7500 (bp = 128C, viscosity = 0.8 cSt, d = 1.61), Fluorinert FC-40 (bp = 155 °C, viscosity = 1.8 cSt, d = 1.85), Fluorinert FC-43 (bp = 174 °C, viscosity = 2.5 cSt, d = 1.86) (both from 3M). In general, selection of perfluorinated filler fluids is based on kinematic viscosity (< 7 cSt for example), and on boiling point (> 150 °C, for example, for use in DNA/RNA-based applications (PCR, etc.)). Filler fluids may, for example, be mixed with surfactants or other additives.

For example, additives may be selected to improve droplet operations and/or reduce loss of reagent or target substances from droplets, formation of microdroplets, cross contamination between droplets, contamination of droplet actuator surfaces, degradation of droplet actuator materials, etc. Compositions of the filler fluid, including surfactant doping, may be selected for improved performance with reagents used in the specific assay protocols and to have an effective interaction (or non-interaction) with droplet actuator materials. Examples of filler fluids and filler fluid formulations suitable for use with the methods and apparatus set forth herein are provided in Srinivasan et al, International Patent Pub. No. WO/2010/027894, entitled "Droplet Actuators, Modified Fluids and Methods," published on June 3, 2010; Srinivasan et al, International Patent Pub. No. WO/2009/021173, entitled "Use of Additives for Enhancing Droplet Operations," published on February 12, 2009; Sista et al., International Patent Pub. No. WO/2008/098236, entitled "Droplet Actuator Devices and Methods Employing Magnetic Beads," published on January 15, 2009; and Monroe et al., U.S. Patent Pub. No. 20080283414, entitled "Electrowetting Devices," published on November 20, 2008. Fluorinated oils may in some cases be doped with fluorinated surfactants, e.g., Zonyl FSO-100 (Sigma-Aldrich) and/or others. A filler fluid may be a liquid. A filler gas can be used instead of a liquid.

Microfluidic devices for biomedical applications include a filler fluid comprising a polymer solubilized in an oil, such as silicon oil. Different types and concentrations of polymer may be mixed with the oil to form a filler fluid that provides a target surface tension with droplets of aqueous buffer. Also discussed herein are methods of adjusting the surface tension between the filler fluid and the buffer to form sample droplets to be within a target range of surface tension. In cases where the filler fluid includes poly(dimethyl)siloxane (PDMS), the chosen surfactant may be soluble in PDMS, but not in the aqueous buffer that contains the sample. One class of polymers, siloxane based block co-polymers, has been identified as soluble in PDMS and therefore useful in microfluidic devices for biomedical applications. Some embodiments of the present application are directed to filler fluids comprising a siloxane based block co-polymer which have a desirable interfacial tension (IFT).

As used herein, IFT refers to the surface tension between liquid phases, such as between the filler fluid and the aqueous liquid (such as buffer) contained within the droplet. In some embodiments, the IFT is measured by dispersing droplets containing certain reagents, e.g., buffers for biomedical applications, in a filler fluid. It should be appreciated that for different reagents, the desirable IFT or IFT range, may vary. Therefore, the filler fluids disclosed herein may be adjusted to achieve a desirable IFT value or IFT range for a certain reagent. The adjustment of the IFT of a filler fluid may be performed in a variety of ways, such as, but not limited to, varying the concentration of a siloxane based block co-polymer solubilized in the filler fluid. For example, the filler fluids disclosed herein may have an interfacial tension (IFT) that is about 3 dynes/cm, about 4 dynes/cm, about 5 dynes/cm, about 6 dynes/cm, about 7 dynes/cm, about 8 dynes/cm, about 9 dynes/cm, about 10 dynes/cm, about 11 dynes/cm, about 12 dynes/cm, about 13 dynes/cm, about 14 dynes/cm, about 15 dynes/cm, about 16 dynes/cm, about 17 dynes/cm, about 18 dynes/cm, about 19 dynes/cm, about 20 dynes/cm. The IFT may be greater or lesser than these values, or alternatively in a range that is between any two of the above values. In some cases, the filler fluid has an IFT of 8-15 dynes/cm. In some cases, the filler fluid has an IFT of 6-12 dynes/cm.

Accordingly, a siloxane based block co-polymer may be solubilized in the filler fluid in a range of concentrations so that a desirable IFT or IFT range may be achieved. The siloxane based block co-polymer may be solubilized in the filler fluid in various concentrationsThe siloxane based block co-polymer is about 0.02% w/w to about 0.1% w/w of the filler fluid, and optionally 0.05% w/w of the filler fluid.

The filler fluids allow droplet formation and movement. The filler fluids should not have side effect on most biological function.

### Siloxane Based Block Co-polvmers

The siloxane based block co-polymer is selected from the group consisting of: CMS-626 (35% hydroxyethylene oxypropylmethylsiloxane)-(dimethylsiloxane) co-polymer, 550-650 cSt, CMS-832 ((hydroxyethyleneoxypropylmethylsiloxane)-(3,4-dimethoxyphenylpropyl)methylsiloxane-dimethylsiloxane terpolymer, 1,000-2,000 cSt), DBE 311 (dimethylsiloxane-(30-35% ethylene oxide) block co-polymer, 10 cSt), DBE 411 (dimethylsiloxane-(45-50% ethylene oxide) block co-polymer, 5-10 cSt), ABP-263 (dodecylmethylsiloxane-hydroxypolyalkyleneoxypropyl methylsiloxane co-polymer, 1,000-4,000 cSt), APT-263 ((60-70% dodecylmethylsiloxane)-(30-40% 2-phenylpropylmethylsiloxane) co-polymer, 1,100-1,300 cSt), DMS S31 (silanol terminated polydimethylsiloxane, 1,000 cSt), FMS 736 ((15-20% tridecafluorooctylmethylsiloxane) - (80-85% dimethylsiloxane) co-polymer, 4,00-7,000 cSt), FMS 121 (poly(3,3,3-trifluoropropylmethylsiloxane), 80-120 cSt), FMS 9922 (silanol terminated polytrifluoropropylmethylsiloxane, 150-250c cSt), FMS 9921 (silanol terminated polytrifluoropropylmethylsiloxane, 50-160 cSt), (all from Gelest, Morrisville, PA), 480290 (Poly(dimethylsiloxane), monoglycidyl ether terminated, 65 cSt), 481246 (Poly(dimethylsiloxane), bis(hydroxyalkyl) terminated, 100 cSt), 481963 (Poly(dimethylsiloxane), hydroxy terminated, 750 cSt), 481955 (Poly(dimethylsiloxane), hydroxy terminated, 65 cSt), 481939 (Poly(dimethylsiloxane), hydroxy terminated, 25 cSt) (all from Sigma, St. Louis, MO), .

It should be appreciated that the ratio between the size of the hydrophilic head and the lipophilic tail and the nature of the hydrophilic interaction can be adjusted for every type of reagents or applications. In some embodiments, the siloxane based block co-polymers are bis-silanols having a structure of: 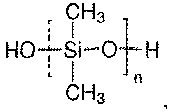 wherein n ≥ 10.

The siloxane based block co-polymers are immiscible with aqueous liquids such as water and other aqueous buffers. In some embodiments, the siloxane based block co-polymers are miscible with oil. In some embodiments, the siloxane based block co-polymers are stable under certain conditions, e.g., thermal, UV, basic, acidic, etc.

### Fluidic Devices

Embodiments disclosed herein include kits comprising fluidic devices comprising a plurality of droplets dispersed in a filler fluid comprising a siloxane block co-polymer. In some cases, the microfluidic device is an electrowetting device. Electrowetting devices and methods of droplet-based actuation by electrowetting are described in U.S. Patent Publication No 2004/0055891.

The microfluidic device may comprise a droplet actuator. The droplet actuator will include a substrate with one or more electrodes arranged for conducting one or more droplet operations. In some cases, the droplet actuator will include one or more arrays, paths or networks of such electrodes. A variety of electrical properties may be employed to effect droplet operations. Examples include electrowetting and electrophoresis.

In some cases, a cartridge is coupled to the droplet actuator. The cartridge may include a top plate, often made of plastic, two hydrophobic coating layers, a dielectric coating layer, and a printed circuit board (PCB) bottom with tracks or paths of electrode in between one hydrophobic layer and the dielectric coating layer. The space or gap between the two hydrophobic layers can be filled with the filler fluids disclosed herein. The droplet movement is triggered by the voltage potential of the cartridge.

### Methods of Conducting Droplet Operation

Droplet operations may be conducted using a variety of mechanisms, including but not limited to, electrowetting, opto-electrowetting, electrostatic, electrophoretic, dielectrophoretic, electro-osmotic, or a combination thereof.

As used herein, the term "droplet operation" means any manipulation of a droplet on a droplet actuator. A droplet operation may, for example, include: loading a droplet into the droplet actuator; dispensing one or more droplets from a source droplet; splitting, separating or dividing a droplet into two or more droplets; transporting a droplet from one location to another in any direction; merging or combining two or more droplets into a single droplet; diluting a droplet; mixing a droplet; agitating a droplet; deforming a droplet; retaining a droplet in position; incubating a droplet; heating a droplet; vaporizing a droplet; cooling a droplet; disposing of a droplet; transporting a droplet out of a droplet actuator; other droplet operations described herein; and/or any combination of the foregoing. The terms "merge," "merging," "combine," "combining" and the like are used to describe the creation of one droplet from two or more droplets. It should be understood that when such a term is used in reference to two or more droplets, any combination of droplet operations that are sufficient to result in the combination of the two or more droplets into one droplet may be used. For example, "merging droplet A with droplet B," can be achieved by transporting droplet A into contact with a stationary droplet B, transporting droplet B into contact with a stationary droplet A, or transporting droplets A and B into contact with each other. The terms "splitting," "separating" and "dividing" are not intended to imply any particular outcome with respect to volume of the resulting droplets (i.e., the volume of the resulting droplets can be the same or different) or number of resulting droplets (the number of resulting droplets may be 2, 3, 4, 5 or more). The term "mixing" refers to droplet operations which result in more homogenous distribution of one or more components within a droplet. Examples of "loading" droplet operations include microdialysis loading, pressure assisted loading, robotic loading, passive loading, and pipette loading.

Droplet operations may be electrode-mediated. In some cases, droplet operations are further facilitated by the use of hydrophilic and/or hydrophobic regions on surfaces and/or by physical obstacles. For examples of droplet operations, see the patents and patent applications cited above under the definition of "droplet actuator." Impedance or capacitance sensing or imaging techniques may sometimes be used to determine or confirm the outcome of a droplet operation. Examples of such techniques are described in Sturmer et al., U.S. Patent Pub. No. 20100194408, entitled "Capacitance Detection in a Droplet Actuator," published on Aug. 5, 2010.

Generally speaking, the sensing or imaging techniques may be used to confirm the presence or absence of a droplet at a specific electrode. For example, the presence of a dispensed droplet at the destination electrode following a droplet dispensing operation confirms that the droplet dispensing operation is effective. Similarly, the presence of a droplet at a detection spot at an appropriate step in an assay protocol may confirm that a previous set of droplet operations has successfully produced a droplet for detection. Droplet transport time can be quite fast.

For example, in various embodiments, transport of a droplet from one electrode to the next may exceed about 1 sec, or about 0.1 sec, or about 0.01 sec, or about 0.001 sec. In one embodiment, the electrode is operated in AC mode but is switched to DC mode for imaging. It is helpful for conducting droplet operations for the footprint area of droplet to be similar to the electrowetting area. Thus, droplets that are 1x-, 2x-or 3x the default droplet volume are usefully operated using 1, 2, and 3 electrodes, respectively. If the droplet footprint is greater than number of electrodes available for conducting a droplet operation at a given time, the difference between the droplet size and the number of electrodes may not be greater than 1. Thus, a 2x droplet may be usefully controlled using one electrode and a 3x droplet may be usefully controlled using two electrodes. When droplets include beads, it is useful for droplet size to be equal to the number of electrodes controlling the droplet, e.g., transporting the droplet.

The manipulation of droplets by a droplet actuator may be electrode mediated, e.g., electrowetting mediated or dielectrophoresis mediated or Coulombic force mediated. Examples of other techniques for controlling droplet operations that may be used in the droplet actuators of the present disclosure include using devices that induce hydrodynamic fluidic pressure, such as those that operate on the basis of mechanical principles (e.g. external syringe pumps, pneumatic membrane pumps, vibrating membrane pumps, vacuum devices, centrifugal forces, piezoelectric/ultrasonic pumps and acoustic forces); electrical or magnetic principles (e.g. electroosmotic flow, electrokinetic pumps, ferrofluidic plugs, electrohydrodynamic pumps, attraction or repulsion using magnetic forces and magnetohydrodynamic pumps); thermodynamic principles (e.g. gas bubble generation/phase-change-induced volume expansion); other kinds of surface-wetting principles (e.g. electrowetting, and optoelectrowetting, as well as chemically, thermally, structurally and radioactively induced surface-tension gradients); gravity; surface tension (e.g., capillary action); electrostatic forces (e.g., electroosmotic flow); centrifugal flow (substrate disposed on a compact disc and rotated); magnetic forces (e.g., oscillating ions causes flow); magnetohydrodynamic forces; and vacuum or pressure differential.

Combinations of two or more of the foregoing techniques may be employed to conduct a droplet operation in a droplet actuator of the present disclosure. Similarly, one or more of the foregoing may be used to deliver liquid into a droplet operations gap, e.g., from a reservoir in another device or from an external reservoir of the droplet actuator (e.g., a reservoir associated with a droplet actuator substrate and a flow path from the reservoir into the droplet operations gap).

Droplet operations surfaces of certain droplet actuators of the present disclosure may be made from hydrophobic materials or may be coated or treated to make them hydrophobic. For example, in some cases some portion or all of the droplet operations surfaces may be derivatized with low surface-energy materials or chemistries, e.g., by deposition or using in situ synthesis using compounds such as poly- or per-fluorinated compounds in solution or polymerizable monomers. Examples include TEFLON^{®} AF (available from DuPont, Wilmington, DE), members of the CYTOP family of materials, coatings in the FLUOROPEL^{®} family of hydrophobic and superhydrophobic coatings (available from Cytonix Corporation, Beltsville, MD), silane coatings, fluorosilane coatings, hydrophobic phosphonate derivatives (e.g., those sold by Aculon, Inc), and NOVEC^{™} electronic coatings (available from 3M Company, St. Paul, MN), other fluorinated monomers for plasma-enhanced chemical vapor deposition (PECVD), and organosiloxane (e.g., SiOC) for PECVD.

Droplet transport voltage and frequency may be selected for performance with reagents used in specific assay protocols. Design parameters may be varied, e.g., number and placement of on-actuator reservoirs, number of independent electrode connections, size (volume) of different reservoirs, placement of magnets/bead washing zones, electrode size, inter-electrode pitch, and gap height (between top and bottom substrates) may be varied for use with specific reagents, protocols, droplet volumes, etc. In some cases, a substrate of the present disclosure may be derivatized with low surface-energy materials or chemistries, e.g., using deposition or in situ synthesis using poly- or per-fluorinated compounds in solution or polymerizable monomers. Examples include TEFLON^{®} AF coatings and FLUOROPEL^{®} coatings for dip or spray coating, other fluorinated monomers for plasma-enhanced chemical vapor deposition (PECVD), and organosiloxane (e.g., SiOC) for PECVD.

Additionally, in some cases, some portion or all of the droplet operations surface may be coated with a substance for reducing background noise, such as background fluorescence from a PCB substrate. For example, the noise-reducing coating may include a black matrix resin, such as the black matrix resins available from Toray industries, Inc., Japan. Electrodes of a droplet actuator may be controlled by a controller or a processor, which is itself provided as part of a system, which may include processing functions as well as data and software storage and input and output capabilities. Reagents may be provided on the droplet actuator in the droplet operations gap or in a reservoir fluidly coupled to the droplet operations gap. The reagents may be in liquid form, e.g., droplets, or they may be provided in a reconstitutable form in the droplet operations gap or in a reservoir fluidly coupled to the droplet operations gap. Reconstitutable reagents may be combined with liquids for reconstitution. An example of reconstitutable reagents suitable for use with the methods and apparatus set forth herein includes those described in Meathrel et al., U.S. Patent No. 7,727,466, entitled "Disintegratable Films for Diagnostic Devices," issued on June 1, 2010.

As used herein, the term "droplet" can mean a volume of liquid on a droplet actuator. In some cases, a droplet is at least partially bounded by the filler fluid. For example, a droplet may be completely surrounded by a filler fluid or may be bounded by filler fluid and one or more surfaces of the droplet actuator. As another example, a droplet may be bounded by filler fluid, one or more surfaces of the droplet actuator, and/or the atmosphere. As yet another example, a droplet may be bounded by filler fluid and the atmosphere. Droplets may, for example, be aqueous or non-aqueous or may be mixtures or emulsions including aqueous and non-aqueous components. Droplets may contain solid particles such as magnetic beads.

Droplets may take a wide variety of shapes. For example include generally disc shaped, slug shaped, truncated sphere, ellipsoid, spherical, partially compressed sphere, hemispherical, ovoid, cylindrical, combinations of such shapes, and various shapes formed during droplet operations, such as merging or splitting or formed as a result of contact of such shapes with one or more surfaces of a droplet actuator. For examples of droplet fluids that may be subjected to droplet operations using the approach of the present disclosure, see Eckhardt et al., International Patent Pub. No. WO/2007/120241, entitled, "Droplet-Based Biochemistry," published on October 25, 2007.

A droplet may include a biological sample, such as whole blood, lymphatic fluid, serum, plasma, sweat, tear, saliva, sputum, cerebrospinal fluid, amniotic fluid, seminal fluid, vaginal excretion, serous fluid, synovial fluid, pericardial fluid, peritoneal fluid, pleural fluid, transudates, exudates, cystic fluid, bile, urine, gastric fluid, intestinal fluid, fecal samples, liquids containing single or multiple cells, liquids containing organelles, fluidized tissues, fluidized organisms, liquids containing multi-celled organisms, biological swabs and biological washes. Moreover, a droplet may include a reagent, such as water, deionized water, saline solutions, acidic solutions, basic solutions, detergent solutions and/or buffers.

A droplet can include nucleic acids, such as DNA, genomic DNA, RNA, mRNA or analogs thereof; nucleotides such as deoxyribonucleotides, ribonucleotides or analogs thereof such as analogs having terminator moieties such as those described in Bentley et al., Nature 456:53-59 (2008); Gormley et al., International Patent Pub. No. WO/2013/131962, entitled, "Improved Methods of Nucleic Acid Sequencing," published on September 12, 2013; Barnes et al., U.S. Patent No. 7,057,026, entitled "Labelled Nucleotides," issued on June 6, 2006; Kozlov et al., International Patent Pub. No. WO/2008/042067, entitled, "Compositions and Methods for Nucleotide Sequencing," published on April 10, 2008; Rigatti et al., International Patent Pub. No. WO/2013/117595, entitled, "Targeted Enrichment and Amplification of Nucleic Acids on a Support," published on August 15, 2013; Hardin et al., U.S. Patent No. 7,329,492, entitled "Methods for Real-Time Single Molecule Sequence Fetermination," issued on February 12, 2008; Hardin et al., U.S. Patent No. 7,211,414, entitled "Enzymatic Nucleic Acid Synthesis: Compositions and Methods for Altering Monomer Incorporation Fidelity," issued on May 1, 2007; Turner et al., U.S. Patent No. 7,315,019, entitled "Arrays of Optical Confinements and Uses Thereof," issued on January 1, 2008; Xu et al., U.S. Patent No. 7,405,281, entitled "Fluorescent Nucleotide Analogs and Uses Therefor," issued on July 29, 2008; and Ranket al., U.S. Patent Pub. No. 20080108082, entitled "Polymerase Enzymes and Reagents for Enhanced Nucleic Acid Sequencing," published on May 8, 2008; enzymes such as polymerases, ligases, recombinases, or transposases; binding partners such as antibodies, epitopes, streptavidin, avidin, biotin, lectins or carbohydrates; or other biochemically active molecules. Other examples of droplet contents include preparing reagents, such as a reagent for a biochemical protocol, such as a nucleic acid amplification protocol, an affinity-based assay protocol, an enzymatic assay protocol, a polynucleotide sequencing reaction, and/or a protocol for analyses of biological fluids. In one example, a polynucleotide sequencing reaction is a process carried out on a nucleotide sequencing machine, such as a next-generation sequencer, for determining the sequence of nucleotides in a polynucleotide fragment. As used herein, a droplet may include one or more beads.

In some cases, the sample or reagent droplet is an aqueous-based. In others, the sample or reagent droplet comprises a mixture of water and one or more organic solvents such as alcoholic solvents. In some others, the sample or reagent droplet contains only one or more organic solvents. In yet others, the droplet comprises a biological sample, such as nucleic acid.

The methods of conducting droplet operation in a microfluidic device provided herein may be used for a variety of biomedical applications, such as nucleic acid amplification protocols, affinity-based assay protocols, sequencing protocols, and protocols for analyses of biological fluids, etc.

### Kits

The present invention includes kits comprising a container comprising a filler fluid comprising a siloxane block co-polymer and a filler fluid, and one or more fluidics devices. In one embodiment the fluidics device is an electrowetting device. The filler fluid may include a siloxane block co-polymer and silicone oil. The silicone oil may be PDMS. The kit may also include a container comprising an aqueous buffer, and the aqueous buffer may be substantially immiscible with the siloxane block co-polymer. Less than about 0.1% of the volume fraction of the silicon block co-polymer in the filler fluid is miscible with the aqueous buffer.
The siloxane block co-polymer is selected from the group consisting of dimethylsiloxane-(45-50% ethylene oxide) block co-polymer,
dimethylsiloxane-(30-35% ethylene oxide) block co-polymer,
35% hydroxyethylene oxypropylmethylsiloxane)-(dimethylsiloxane) co-polymer,
(hydroxyethyleneoxypropylmethylsiloxane)-(3,4-dimethoxyphenylpropyl)methylsiloxane-dimethylsiloxane terpolymer,
poly(dimethylsiloxane), monoglycidyl ether terminated,
poly(dimethylsiloxane), bis(hydroxyalkyl) terminated,
Poly(dimethylsiloxane), hydroxy terminated,
monocarbinol terminated polydimethylsiloxane, asymmetric,
dodecylmethylsiloxane-hydroxypolyalkyleneoxypropyl methylsiloxane co-polymer,
(60-70% dodecylmethylsiloxane)-(30-40% 2-phenylpropylmethylsiloxane) co-polymer, 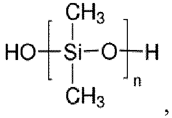 wherein n ≥ 10,
poly(dimethylsiloxane), hydroxy terminated,
silanol terminated polytrifluoropropylmethylsiloxane,
(15-20% tridecafluorooctylmethylsiloxane) - (80-85% dimethylsiloxane) co-polymer,
   poly(3,3,3-trifluoropropylmethylsiloxane). In some embodiments, the concentration of the siloxane block co-polymer in the filler fluid is about 0.02% w/w to about 0.1% w/w. In some embodiments, the concentration of the siloxane block co-polymer in the filler fluid is about 0.05% w/w. In some embodiments, the filler fluid comprises polydimethylsiloxane (PDMS).

### Methods of Loading Filler Fluid

Also disclosed but not part of the invention are methods of loading a filler fluid into a microfluidic device comprising a plurality of droplets dispersed in a filler fluid comprising a siloxane block co-polymer solubilized in the filler fluid. The surface tension between the droplets and the filler fluid is between 3 and 10-12 dynes/cm. The methods comprise moving the droplets using a mechanism selected from electrowetting, opto-electrowetting, electrostatic, electrophoretic, dielectrophoretic, electro-osmotic, or a combination thereof.

### EXAMPLES

### Example 1 - Not in accordance with the invention

Multiple siloxane block co-polymer dimethicones were obtained, including several from Momentive (Waterford, NY), such as Silsoft 900 and SF 1528. Another siloxane block co-polymer, known as CMS-222, was obtained from Gelest (Morrisville, PA). CMS-222 is a (hydroxypropyleneoxypropyl) methylsiloxane - dimethylsiloxane co-polymer. First, the solubility of these siloxane block co-polymers, and others, were tested in polydimethylsiloxane (PDMS) silicon oil at 1%. Siloxane block co-polymers that were insoluble in PDMS oil were removed from the list. Second, the insolubility in water of these siloxane block co-polymers at 1% was also verified. Soluble siloxane block co-polymers were also removed from the list. Finally the surface tension of water, using the pendant drop method, was tested for the proper concentration of surfactant to achieve the right target level of surface tension. Dimethicones that failed to bring surface tension of oil/water down to about 10 dynes/cm or less were removed from the list. As used herein, a first liquid is soluble in another liquid when the first liquid completely dissolves in the other liquid.

Finally the PDMS oil, spiked with one of these dimethicones, was introduced in a Neoprep^{™} microassay cartridge, commercially available from Illumina, San Diego, USA. The Neoprep^{™} cartridge is an electrowetting assay cartridge composed of a bottom plate made from a PCB coated with a dielectric and a hydrophobic layer and a top plate composed of a polymer (polycarbonate) coated with a hydrophobic layer. It was verified that droplets of water could be formed and moved across the surfaces within this filler fluid combination. It was found that filler oil spiked with dimethicone, particularly CMS-222 from Gelest, was found to operate within the cartridge well. Additional testing with Silsoft 900 surfactant also demonstrated that adding a dimethicone surfactant to the oil allowed the device to work very well.

### Example 2 - Not in accordance with the invention

Formulation procedure: An empty 5L bottle was placed on a scale and Tare weight. 4L of 5cSt PDMS oil was added into the 5L bottle, and bare oil weight was recorded. The Tare weight of bare oil was recorded before adding the silicon block co-polymer CMS-222. 2.0 grams of CMS-222 was added to the 4L of oil, and actual CMS-222 weight was recorded. If the percentage was not within an acceptable range, extra 5cSt PDMS oil was added to meet the concentration specifications and the calculation was repeated.

A 3" magnetic stir bar was placed inside the bottle, and the bottle was capped and moved to a stir plate. The stir speed was set to ∼1½ (vortex occupies about half the solution) and mixed for 1 hour at room temperature. The solution was then transferred into small glass vials, at 8.2 mL dispense volume per vial, and degassed for 3 hours under vacuum (< 1 Torr). The target concentration of 0.05% CMS-222 within PDMS was achieved.

### Example 3 - Not in accordance with the invention

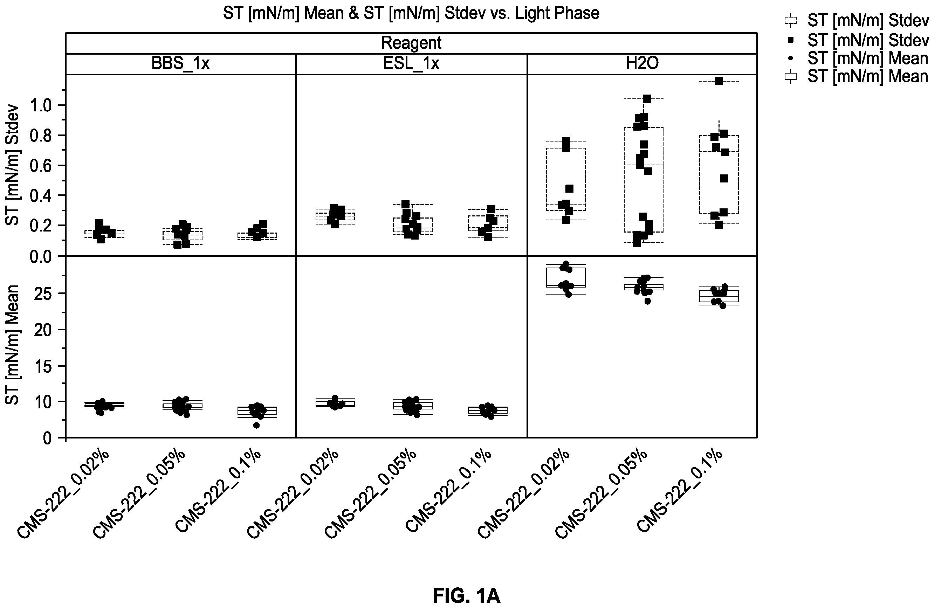

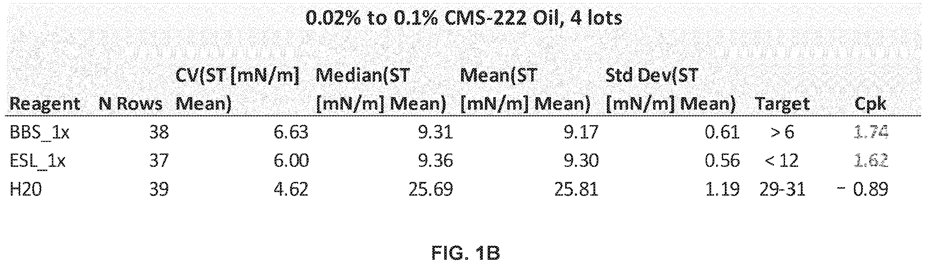

Interfacial tension (IFT) for filler fluids having concentrations of 0.02% to 0.1% CMS-222 in 5cSt PDMS oil were tested against different reagents and water. **FIG. 1A** shows the IFT data resulting from these tests. As shown in **FIG. 1A****,** varying concentrations of CMS-222 were tested against two aqueous buffers (BBS and ESL) and water to determine if the resulting surface tension would be within a target surface tension range. **FIG. 1B** reports the results of the tests from **FIG. 1A****,** and shows the ability of buffers BBS and ESL within filler fluids containing CMS-222 to maintain a surface tension between 6-12 mN/m. As shown, significant IFT improvement was achieved with filler fluids that had CMS-222 in comparison to filler fluids containing Span^{®} 85 (data not shown), and the process is capable, having a process capability index of cpk > 1.33 with a tested concentration range (0.02-0.1%).

### Example 4 - Not in accordance with the invention

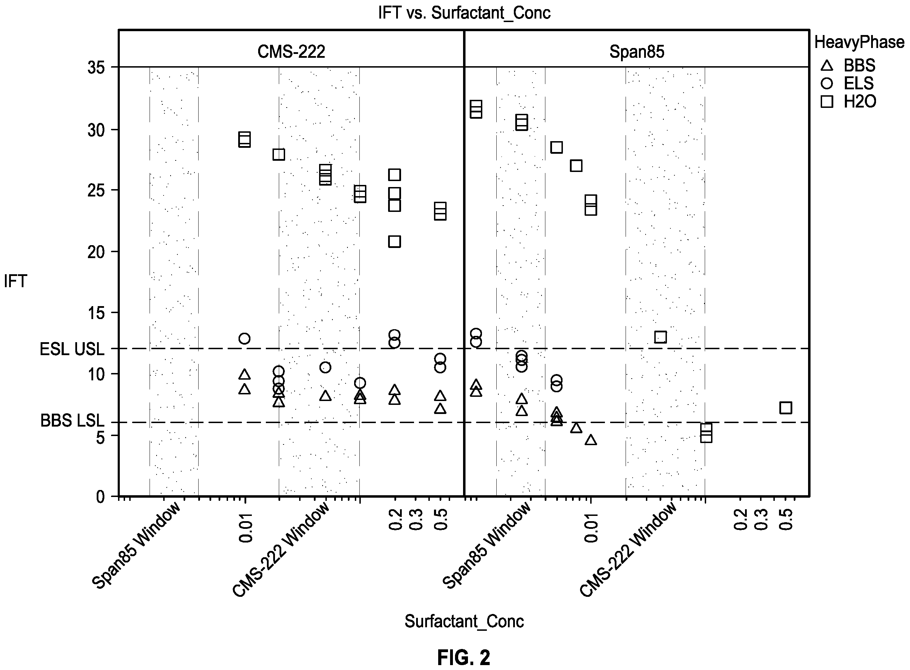

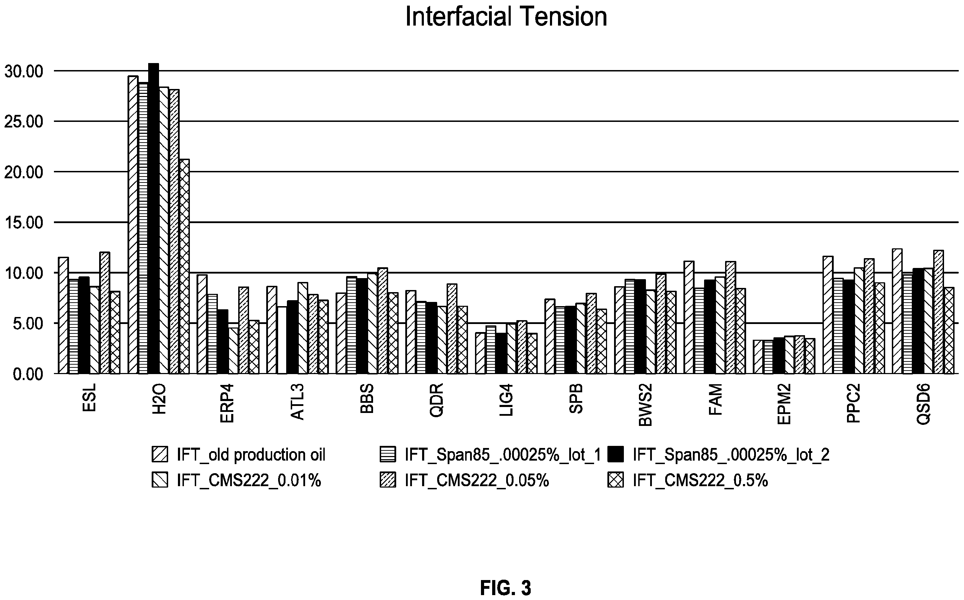

IFT working ranges of CMS-222 and Span^{®} 85 (Sigma, St. Louis, MO) were tested in reagent A (BBS), reagent B (ESL), and other buffers ERP4, ATL3, QDR, LIG4, SPB, BWS2, FAM, EPM2, PPC2, QSD6 and water. Standard IFT measurements using a pendant drop analysis in oil with CMS 222 and Span^{®} 85 were used. A drop of reagent was pushed out of a syringe needle, into a container with the PDMS oil, until it almost detached from the needle tip. At this point an image was snapped of the hanging drop and its contour profile was analyzed for its shape via the Laplace equation, giving the IFT. The IFT working range was determined by prior functional correlation: < 12 dynes/cm for reagent B and > 6 dynes/cm for reagent A. **FIG. 2** is a diagram that shows experimental data on the measured IFT of CMS-222 and Span^{®} 85 vs. various concentrations of surfactants (shown in logarithmic scale). These data demonstrate that the working range, shown as a Window, for Span^{®} 85 is 0.0015% - 0.004% w/w, and the working range for CMS-222 is 0.02% - 0.1% w/w. IFT of CMS-222 was also tested against a variety of standard Nano reagents. **FIG. 3** shows that the IFT of CMS-222 against all buffers was very similar to those of old and current standard production oil.

### Example 5

A number of siloxane block co-polymers were tested for their capacity to form micelles or aggregates. They were also tested to determine their range of working concentration, miscibility with water. Even if the co-polymers were only slightly miscible in water they were ruled out. The co-polymers were also tested for their miscibility with the PDMS oil (on a scale of good, pass, bad, fail). Additional factors tested for each of the different co-polymers included their ease of use, handling or preparation (manufacturing friendly). Other factors included their availability, cost, toxicity, flammability and stability (thermal, UV, base, acid, or reagents). We found that siloxane-based surfactants, particularly dimethicones, met all those criteria for use as a block co-polymer within a filler fluid for fluidics devices.

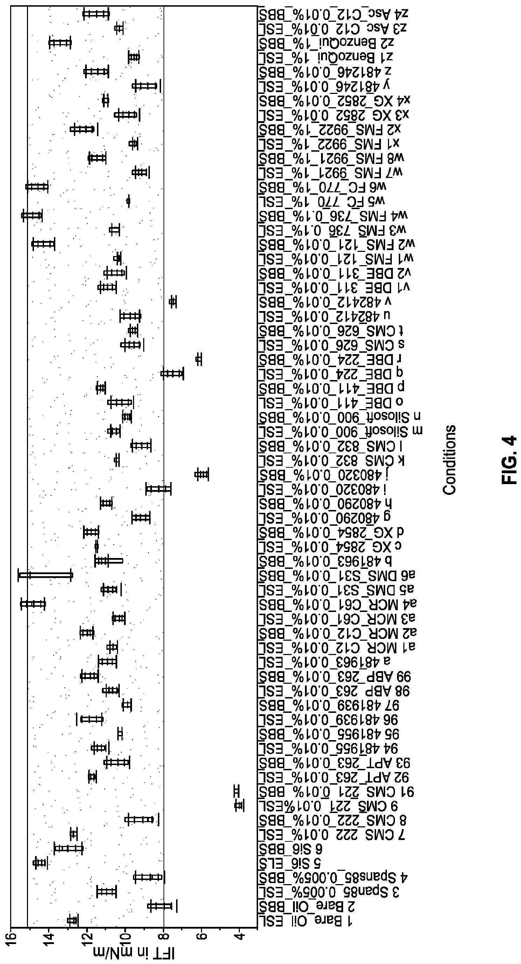

Then, the siloxane block co-polymers were tested for surface tension by measuring IFT at 0.01% against ESL and BBS buffers. The co-polymers that recorded an IFT of about 8-15 mN/m were selected as candidates for use as surfactants. The test results are summarized in **FIG. 4** and Table 1 below. It was discovered that some surfactants, mainly fluoro-derivatives, increased the IFT of BBS and reduced the IFT of ESL. This inverted the trend compared to bare oil.

CMS-221 ((carbinol functional) methylsiloxane - dimethylsiloxane co-polymer, 125-150 cSt); MCR C61 (monodicarbinol terminated polydimethylsiloxane, asymmetric, 50-60 cSt); DBE-224 (dimethylsiloxane-(25-30% ethylene oxide) block co-polymer, 400 cSt), and 480320 (Poly[dimethylsiloxane-co-[3-(2-(2-hydroxyethoxy)ethoxy)propyl]methylsiloxane], 75 cSt) are included in Table 1 as comparative examples.

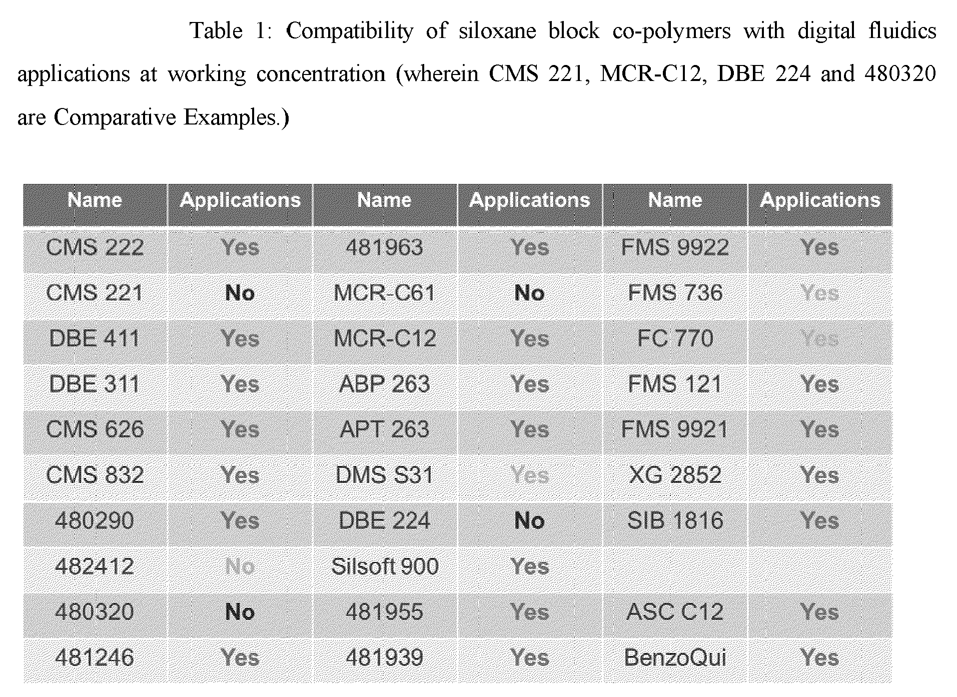

## Claims

1. A filler fluid for a fluidic device, comprising:
a silicone oil which is or comprises polydimethylsiloxane (PDMS) ; and
a siloxane block co-polymer solubilized in the silicone oil, wherein less than 0.1% of the volume fraction of the siloxane block co-polymer in the filler fluid is miscible with an aqueous liquid selected from water and other aqueous buffers; and further, wherein the siloxane block co-polymer is selected from the group consisting of:
dimethylsiloxane-(45-50% ethylene oxide) block co-polymer,
dimethylsiloxane-(30-35% ethylene oxide) block co-polymer,
(35% hydroxyethylene oxypropylmethylsiloxane)-(dimethylsiloxane) co-polymer, (hydroxyethyleneoxypropylmethylsiloxane)-(3,4-dimethoxyphenylpropyl)methylsiloxane-dimethylsiloxane terpolymer,
poly(dimethylsiloxane), monoglycidyl ether terminated,
poly(dimethylsiloxane), bis(hydroxyalkyl) terminated,
Poly(dimethylsiloxane), hydroxy terminated,
monocarbinol terminated polydimethylsiloxane, asymmetric,
dodecylmethylsiloxane-hydroxypolyalkyleneoxypropyl methylsiloxane co-polymer,
(60-70% dodecylmethylsiloxane)-(30-40% 2-phenylpropylmethylsiloxane) co-polymer, 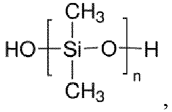 wherein n ≥ 10,
poly(dimethylsiloxane), hydroxy terminated,
silanol terminated polytrifluoropropylmethylsiloxane,
(15-20% tridecafluorooctylmethylsiloxane) - (80-85% dimethylsiloxane) co-polymer, poly(3,3,3-trifluoropropylmethylsiloxane).

2. The filler fluid of Claim 1, wherein the concentration of the siloxane block co-polymer in the filler fluid is 0.02% w/w to 0.1% w/w, optionally wherein the concentration of the siloxane block co-polymer in the filler fluid is 0.05% w/w.

3. The filler fluid of Claim 1, wherein the silicone oil comprises polydimethylsiloxane (PDMS)

4. The filler fluid of Claim 1, wherein the surface tension of the filler fluid and a droplet of aqueous liquid is between 0.003 N/m and 0.012 N/m (3 and 12 dynes/cm).

5. A kit comprising:
a fluidics device; and
a container comprising a filler fluid, wherein the filler fluid comprises:
a silicone oil which is or comprises polydimethylsiloxane (PDMS); and
a siloxane block co-polymer solubilized in the silicone oil, wherein less than 0.1% of the volume fraction of the siloxane block co-polymer in the filler fluid is miscible with an aqueous liquid selected from water and other aqueous buffers; and
further wherein the siloxane block co-polymer is selected from the group consisting of:
dimethylsiloxane-(45-50% ethylene oxide) block co-polymer,
dimethylsiloxane-(30-35% ethylene oxide) block co-polymer,
(35% hydroxyethylene oxypropylmethylsiloxane)-(dimethylsiloxane) co-polymer, (hydroxyethyleneoxypropylmethylsiloxane)-(3,4-dimethoxyphenylpropyl)methylsiloxane-dimethylsiloxane terpolymer,
poly(dimethylsiloxane), monoglycidyl ether terminated,
poly(dimethylsiloxane), bis(hydroxyalkyl) terminated,
Poly(dimethylsiloxane), hydroxy terminated,
monocarbinol terminated polydimethylsiloxane, asymmetric,
dodecylmethylsiloxane-hydroxypolyalkyleneoxypropyl methylsiloxane co-polymer,
(60-70% dodecylmethylsiloxane)-(30-40% 2-phenylpropylmethylsiloxane) co-polymer, 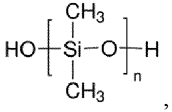 wherein n ≥ 10,
poly(dimethylsiloxane), hydroxy terminated,
silanol terminated polytrifluoropropylmethylsiloxane,
(15-20% tridecafluorooctylmethylsiloxane) - (80-85% dimethylsiloxane) co-polymer, poly(3,3,3-trifluoropropylmethylsiloxane).

6. The kit of Claim 5, further comprising a container comprising said aqueous buffer.

7. The kit of Claim 5, wherein the silicone oil comprises polydimethylsiloxane (PDMS).

8. The kit of Claim 5, wherein the fluidics device is an electrowetting, opto-electrowetting, electrostatic, electrophoretic, dielectrophoretic, or electroosmotic device.

9. A method of conducting droplet operations in a fluidic device comprising:
moving a plurality of aqueous droplets through a filler fluid within a fluidic device, wherein the filler fluid comprises:
a silicone oil which is or comprises polydimethylsiloxane (PDMS);
and a siloxane block co-polymer solubilized in the silicone oil, wherein less than 0.1% of the volume fraction of the siloxane block co-polymer in the filler fluid is miscible with an aqueous liquid selected from water and other aqueous buffers; and
further wherein the siloxane block co-polymer selected from the group consisting of: dimethylsiloxane-(45-50% ethylene oxide) block co-polymer,
dimethylsiloxane-(30-35% ethylene oxide) block co-polymer,
(35% hydroxyethylene oxypropylmethylsiloxane)-(dimethylsiloxane) co-polymer, (hydroxyethyleneoxypropylmethylsiloxane)-(3,4-dimethoxyphenylpropyl)methylsiloxane-dimethylsiloxane terpolymer,
poly(dimethylsiloxane), monoglycidyl ether terminated,
poly(dimethylsiloxane), bis(hydroxyalkyl) terminated,
Poly(dimethylsiloxane), hydroxy terminated,
monocarbinol terminated polydimethylsiloxane, asymmetric,
dodecylmethylsiloxane-hydroxypolyalkyleneoxypropyl methylsiloxane co-polymer,
(60-70% dodecylmethylsiloxane)-(30-40% 2-phenylpropylmethylsiloxane) co-polymer, 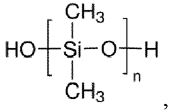 wherein n ≥ 10,
poly(dimethylsiloxane), hydroxy terminated,
silanol terminated polytrifluoropropylmethylsiloxane,
(15-20% tridecafluorooctylmethylsiloxane) - (80-85% dimethylsiloxane) co-polymer, poly(3,3,3-trifluoropropylmethylsiloxane), optionally
wherein moving the plurality of aqueous droplets comprises using electrowetting, opto-electrowetting, electrostatic, electrophoretic, dielectrophoretic, or electroosmotic device, or a combination thereof, to move the plurality of aqueous droplets.

10. The method of Claim 9, wherein the surface tension between the plurality of aqueous droplets and the filler fluid is between 0.003 N/m and 0.012 N/m (3 and 12 dynes/cm).

11. The method of Claim 9, wherein moving the plurality of droplets comprises performing polymerase chain reaction.

12. The method of Claim 9, wherein moving the plurality of droplets comprises preparing a sample for a polynucleotide sequencing reaction.

## Patentansprüche

1. Ein Füllstofffluid für eine Fluidikvorrichtung, das Folgendes beinhaltet:
ein Silikonöl, das Polydimethylsiloxan (PDMS) ist oder beinhaltet; und
ein Siloxan-Block-Copolymer, das in dem Silikonöl solubilisiert ist, wobei weniger als 0,1 % der Volumenfraktion des Siloxan-Block-Copolymers in dem Füllstofffluid mit einer wässrigen Flüssigkeit, ausgewählt aus Wasser und anderen wässrigen Puffern, mischbar ist; und wobei ferner das Siloxan-Block-Copolymer ausgewählt ist aus der Gruppe, bestehend aus:
Dimethylsiloxan(45-50 % Ethylenoxid)-Block-Copolymer,
Dimethylsiloxan(30-35 % Ethylenoxid)-Block-Copolymer,
(35 % Hydroxyethylenoxypropylmethylsiloxan)-(Dimethylsiloxan)-Copolymer, (Hydroxyethylenoxypropylmethylsiloxan)-(3,4-Dimethoxyphenylpropyl)methylsiloxandimethylsiloxan-Terpolymer, Poly(dimethylsiloxan), monoglycidyletherterminiert,
Poly(dimethylsiloxan), bis(hydroxyalkyl)terminiert,
Poly(dimethylsiloxan), hydroxyterminiert,
monocarbinolterminiertem Polydimethylsiloxan, asymmetrisch,
Dodecylmethylsiloxan-Hydroxypolyalkylenoxypropylmethylsiloxan-Copolymer, (60-70 % Dodecylmethylsiloxan)-(30-40 % 2-Phenylpropylmethylsiloxan)-Copolymer, 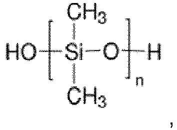 wobei n ≥ 10,
Poly(dimethylsiloxan), hydroxyterminiert,
silanolterminiertem Polytrifluorpropylmethylsiloxan,
(15-20 % Tridecafluoroctylmethylsiloxan) - (80-85 % Dimethylsiloxan)-Copolymer, Poly(3,3,3-Trifluorpropylmethylsiloxan).

2. Füllstofffluid gemäß Anspruch 1, wobei die Konzentration des Siloxan-Block-Copolymers in dem Füllstofffluid 0,02 Gew.-% bis 0,1 Gew.-% beträgt, wobei optional die Konzentration des Siloxan-Block-Copolymers in dem Füllstofffluid 0,05 Gew.-% beträgt.

3. Füllstofffluid gemäß Anspruch 1, wobei das Silikonöl Polydimethylsiloxan (PDMS) beinhaltet.

4. Füllstofffluid gemäß Anspruch 1, wobei die Oberflächenspannung des Füllstofffluids und eines Tröpfchens einer wässrigen Flüssigkeit zwischen 0,003 N/m und 0,012 N/m (3 und 12 dyn/cm) beträgt.

5. Ein Kit, das Folgendes beinhaltet:
eine Fluidikvorrichtung; und
einen Behälter, der ein Füllstofffluid beinhaltet, wobei das Füllstofffluid Folgendes beinhaltet:
ein Silikonöl, das Polydimethylsiloxan (PDMS) ist oder beinhaltet; und
ein Siloxan-Block-Copolymer, das in dem Silikonöl solubilisiert ist, wobei weniger als 0,1 % der Volumenfraktion des Siloxan-Block-Copolymers in dem Füllstofffluid mit einer wässrigen Flüssigkeit, ausgewählt aus Wasser und anderen wässrigen Puffern,
mischbar ist; und
wobei ferner das Siloxan-Block-Copolymer ausgewählt ist aus der Gruppe, bestehend aus:
Dimethylsiloxan(45-50 % Ethylenoxid)-Block-Copolymer,
Dimethylsiloxan(30-35 % Ethylenoxid)-Block-Copolymer,
(35 % Hydroxyethylenoxypropylmethylsiloxan)-(Dimethylsiloxan)-Copolymer, (Hydroxyethylenoxypropylmethylsiloxan)-(3,4-Dimethoxyphenylpropyl)methylsiloxandimethylsiloxan-Terpolymer, Poly(dimethylsiloxan), monoglycidyletherterminiert,
Poly(dimethylsiloxan), bis(hydroxyalkyl)terminiert,
Poly(dimethylsiloxan), hydroxyterminiert,
monocarbinolterminiertem Polydimethylsiloxan, asymmetrisch, Dodecylmethylsiloxan-Hydroxypolyalkylenoxypropylmethylsiloxan-Copolymer,
(60-70 % Dodecylmethylsiloxan)-(30-40 % 2-Phenylpropylmethylsiloxan)-Copolymer, 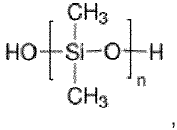 wobei n ≥ 10,
Poly(dimethylsiloxan), hydroxyterminiert,
silanolterminiertem Polytrifluorpropylmethylsiloxan,
(15-20 % Tridecafluoroctylmethylsiloxan) - (80-85 % Dimethylsiloxan)-Copolymer, Poly(3,3,3-Trifluorpropylmethylsiloxan).

6. Kit gemäß Anspruch 5, das ferner einen Behälter beinhaltet, der den wässrigen Puffer beinhaltet.

7. Kit gemäß Anspruch 5, wobei das Silikonöl Polydimethylsiloxan (PDMS) beinhaltet.

8. Kit gemäß Anspruch 5, wobei die Fluidikvorrichtung eine Elektrobenetzungs-, Optoelektrobenetzungs-, Elektrostatik-, Elektrophorese-, Dielektrophorese-, Elektroosmotikvorrichtung ist.

9. Ein Verfahren zum Ausführen von Tröpfchenvorgängen in einer Fluidikvorrichtung, das Folgendes beinhaltet:
Bewegen einer Vielzahl von wässrigen Tröpfchen durch ein Füllstofffluid innerhalb einer Fluidikvorrichtung, wobei das Füllstofffluid Folgendes beinhaltet:
ein Silikonöl, das Polydimethylsiloxan (PDMS) ist oder beinhaltet;
und ein Siloxan-Block-Copolymer, das in dem Silikonöl solubilisiert ist, wobei weniger als 0,1 % der Volumenfraktion des Siloxan-Block-Copolymers in dem Füllstofffluid mit einer wässrigen Flüssigkeit, ausgewählt aus Wasser und anderen wässrigen Puffern, mischbar ist; und
wobei ferner das Siloxan-Block-Copolymer ausgewählt ist aus der Gruppe, bestehend aus:
Dimethylsiloxan(45-50 % Ethylenoxid)-Block-Copolymer,
Dimethylsiloxan(30-35 % Ethylenoxid)-Block-Copolymer,
(35 % Hydroxyethylenoxypropylmethylsiloxan)-(Dimethylsiloxan)-Copolymer, (Hydroxyethylenoxypropylmethylsiloxan)-(3,4-Dimethoxyphenylpropyl)methylsiloxandimethylsiloxan-Terpolymer, Poly(dimethylsiloxan), monoglycidyletherterminiert,
Poly(dimethylsiloxan), bis(hydroxyalkyl)terminiert,
Poly(dimethylsiloxan), hydroxyterminiert,
monocarbinolterminiertem Polydimethylsiloxan, asymmetrisch, Dodecylmethylsiloxan-Hydroxypolyalkylenoxypropylmethylsiloxan-Copolymer, (60-70 % Dodecylmethylsiloxan)-(30-40 % 2-Phenylpropylmethylsiloxan)-Copolymer, 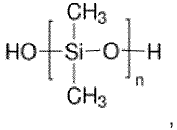 wobei n ≥ 10,
Poly(dimethylsiloxan), hydroxyterminiert,
silanolterminiertem Polytrifluorpropylmethylsiloxan,
(15-20 % Tridecafluoroctylmethylsiloxan) - (80-85 % Dimethylsiloxan)-Copolymer, Poly(3,3,3-Trifluorpropylmethylsiloxan),
wobei optional das Bewegen der Vielzahl von wässrigen Tröpfchen das Verwenden eine Elektrobenetzungs-, Optoelektrobenetzungs-, Elektrostatik-, Elektrophorese-, Dielektrophorese- oder Elektroosmotikvorrichtung oder einer Kombination davon beinhaltet, um die Vielzahl von wässrigen Tröpfchen zu bewegen.

10. Verfahren gemäß Anspruch 9, wobei die Oberflächenspannung zwischen der Vielzahl von wässrigen Tröpfchen und dem Füllstofffluid zwischen 0,003 N/m und 0,012 N/m (3 und 12 dyn/cm) beträgt.

11. Verfahren gemäß Anspruch 9, wobei das Bewegen der Vielzahl von Tröpfchen das Durchführen einer Polymerasekettenreaktion beinhaltet.

12. Verfahren gemäß Anspruch 9, wobei das Bewegen der Vielzahl von Tröpfchen das Vorbereiten einer Probe für eine Polynukleotid-Sequenzierungsreaktion beinhaltet.

## Revendications

1. Un fluide de remplissage pour un dispositif fluidique, comprenant :
une huile de silicone qui est ou comprend du polydiméthylsiloxane (PDMS) ; et
un copolymère à blocs siloxane solubilisé dans l'huile de silicone, dans lequel moins de 0,1 % de la fraction volumique du copolymère à blocs siloxane dans le fluide de remplissage est miscible à un liquide aqueux sélectionné parmi l'eau et d'autres tampons aqueux ; et en outre, dans lequel le copolymère à blocs siloxane est sélectionné dans le groupe constitué :
d'un copolymère à blocs diméthylsiloxane - (45 à 50 % d'oxyde d'éthylène),
d'un copolymère à blocs diméthylsiloxane - (30 à 35 % d'oxyde d'éthylène),
d'un copolymère de (35 % d'hydroxyéthylène
oxypropylméthylsiloxane) - (diméthylsiloxane),
d'un terpolymère d'(hydroxyéthylène oxypropylméthylsiloxane) - (3,4-diméthoxyphénylpropyl)méthylsiloxane - diméthylsiloxane,
d'un poly(diméthylsiloxane), à terminaison éther monoglycidylique,
d'un poly(diméthylsiloxane), à terminaison bis(hydroxyalkyle),
d'un poly(diméthylsiloxane), à terminaison hydroxy,
d'un polydiméthylsiloxane à terminaison monocarbinol, asymétrique,
d'un copolymère de dodécylméthylsiloxane - hydroxypolyalkylène oxypropylméthylsiloxane,
d'un copolymère de (60 à 70 % de dodécylméthylsiloxane) - (30 à 40 % de 2-phénylpropylméthylsiloxane), 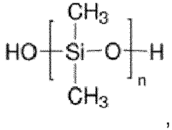 dans lequel n ≥ 10,
d'un poly(diméthylsiloxane), à terminaison hydroxy,
d'un polytrifluoropropylméthylsiloxane à terminaison silanol,
d'un copolymère de (15 à 20 % de tridécafluorooctylméthylsiloxane) - (80 à 85 % de diméthylsiloxane),
de poly(3,3,3-trifluoropropylméthylsiloxane).

2. Le fluide de remplissage de la revendication 1, dans lequel la concentration du copolymère à blocs siloxane dans le fluide de remplissage va de 0,02 % en poids/poids à 0,1 % en poids/poids, facultativement dans lequel la concentration du copolymère à blocs siloxane dans le fluide de remplissage est de 0,05 % en poids/poids.

3. Le fluide de remplissage de la revendication 1, dans lequel l'huile de silicone comprend du polydiméthylsiloxane (PDMS).

4. Le fluide de remplissage de la revendication 1, dans lequel la tension superficielle du fluide de remplissage et une gouttelette de liquide aqueux est comprise entre 0,003 N/m et 0,012 N/m (3 et 12 dynes/cm).

5. Un kit comprenant :
un dispositif fluidique ; et
un récipient comprenant un fluide de remplissage, dans lequel le fluide de remplissage comprend :
une huile de silicone qui est ou comprend du polydiméthylsiloxane (PDMS) ; et
un copolymère à blocs siloxane solubilisé dans l'huile de silicone, dans lequel moins de 0,1 % de la fraction volumique du copolymère à blocs siloxane dans le fluide de remplissage est miscible à un liquide aqueux sélectionné parmi l'eau et d'autres tampons aqueux ; et
en outre dans lequel le copolymère à blocs siloxane est sélectionné dans le groupe constitué :
d'un copolymère à blocs diméthylsiloxane - (45 à 50 % d'oxyde d'éthylène),
d'un copolymère à blocs diméthylsiloxane - (30 à 35 % d'oxyde d'éthylène),
d'un copolymère de (35 % d'hydroxyéthylène
oxypropylméthylsiloxane) - (diméthylsiloxane),
d'un terpolymère d'(hydroxyéthylène oxypropylméthylsiloxane) - (3,4-diméthoxyphénylpropyl)méthylsiloxane - diméthylsiloxane,
d'un poly(diméthylsiloxane), à terminaison éther monoglycidylique,
d'un poly(diméthylsiloxane), à terminaison bis(hydroxyalkyle),
d'un poly(diméthylsiloxane), à terminaison hydroxy,
d'un polydiméthylsiloxane à terminaison monocarbinol, asymétrique,
d'un copolymère de dodécylméthylsiloxane - hydroxypolyalkylène oxypropylméthylsiloxane,
d'un copolymère de (60 à 70 % de dodécylméthylsiloxane) - (30 à 40 % de 2-phénylpropylméthylsiloxane), 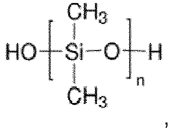 dans lequel n ≥ 10,
d'un poly(diméthylsiloxane), à terminaison hydroxy,
d'un polytrifluoropropylméthylsiloxane à terminaison silanol,
d'un copolymère de (15 à 20 % de tridécafluorooctylméthylsiloxane) - (80 à 85 % de diméthylsiloxane),
de poly(3,3,3-trifluoropropylméthylsiloxane).

6. Le kit de la revendication 5, comprenant en outre un récipient comprenant ledit tampon aqueux.

7. Le kit de la revendication 5, dans lequel l'huile de silicone comprend du polydiméthylsiloxane (PDMS).

8. Le kit de la revendication 5, dans lequel le dispositif fluidique est un dispositif d'électromouillage, d'optoélectromouillage, électrostatique, électrophorétique, diélectrophorétique, ou électroosmotique.

9. Un procédé pour mener des opérations de gouttelettes dans un dispositif fluidique comprenant :
le déplacement d'une pluralité de gouttelettes aqueuses à travers un fluide de remplissage au sein d'un dispositif fluidique, dans lequel le fluide de remplissage comprend :
une huile de silicone qui est ou comprend du polydiméthylsiloxane (PDMS) ;
et un copolymère à blocs siloxane solubilisé dans l'huile de silicone, dans lequel moins de 0,1 % de la fraction volumique du copolymère à blocs siloxane dans le fluide de remplissage est miscible à un liquide aqueux sélectionné parmi l'eau et d'autres tampons aqueux ; et
en outre dans lequel le copolymère à blocs siloxane est sélectionné dans le groupe constitué :
d'un copolymère à blocs diméthylsiloxane - (45 à 50 % d'oxyde d'éthylène),
d'un copolymère à blocs diméthylsiloxane - (30 à 35 % d'oxyde d'éthylène),
d'un copolymère de (35 % d'hydroxyéthylène
oxypropylméthylsiloxane) - (diméthylsiloxane),
d'un terpolymère d'(hydroxyéthylène oxypropylméthylsiloxane) - (3,4-diméthoxyphénylpropyl)méthylsiloxane - diméthylsiloxane,
d'un poly(diméthylsiloxane), à terminaison éther monoglycidylique,
d'un poly(diméthylsiloxane), à terminaison bis(hydroxyalkyle),
d'un poly(diméthylsiloxane), à terminaison hydroxy,
d'un polydiméthylsiloxane à terminaison monocarbinol, asymétrique,
d'un copolymère de dodécylméthylsiloxane - hydroxypolyalkylène oxypropylméthylsiloxane,
d'un copolymère de (60 à 70 % de dodécylméthylsiloxane) - (30 à 40 % de 2-phénylpropylméthylsiloxane), 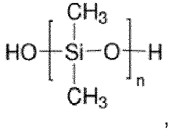 dans lequel n ≥ 10,
d'un poly(diméthylsiloxane), à terminaison hydroxy,
d'un polytrifluoropropylméthylsiloxane à terminaison silanol,
d'un copolymère de (15 à 20 % de tridécafluorooctylméthylsiloxane) - (80 à 85 % de diméthylsiloxane),
de poly(3,3,3-trifluoropropylméthylsiloxane), facultativement dans lequel le déplacement de la pluralité de gouttelettes aqueuses comprend l'utilisation d'un dispositif d'électromouillage, d'optoélectromouillage, électrostatique, électrophorétique, diélectrophorétique, ou électroosmotique, ou d'une combinaison de ceux-ci, afin de déplacer la pluralité de gouttelettes aqueuses.

10. Le procédé de la revendication 9, dans lequel la tension superficielle entre la pluralité de gouttelettes aqueuses et le fluide de remplissage est comprise entre 0,003 N/m et 0,012 N/m (3 et 12 dynes/cm).

11. Le procédé de la revendication 9, dans lequel le déplacement de la pluralité de gouttelettes comprend la réalisation d'une réaction en chaîne par polymérase.

12. Le procédé de la revendication 9, dans lequel le déplacement de la pluralité de gouttelettes comprend la préparation d'un échantillon pour une réaction de séquençage de polynucléotides.
